# EUROPÄISCHE PATENTSCHRIFT

(11) **EP 0 485 564 B1**
(45) Veröffentlichungstag und Bekanntmachung des Hinweises auf die Patenterteilung: **24.08.1994**
(21) Anmeldenummer: 91910307.7
(22) Anmeldetag: 21.05.1991
(51) Int. Cl.: A61B 5/14, G01N 35/02

(54) **VORRICHTUNG ZUR ENTNAHME VON KÖRPERFLÜSSIGKEITEN**
DEVICE FOR WITHDRAWING BODY FLUIDS
DISPOSITIF DE PRELEVEMENT DE FLUIDES CORPORELS

(30) Priorität: 01.06.1990 AT 1208/90
(43) Veröffentlichungstag der Anmeldung: 20.05.1992
(73) Patentinhaber: AVL Medical Instruments AG, 8207 Schaffhausen (CH)
(72) Erfinder: SKRABAL, Falko, A-8010 Graz (AT); KLEINHAPPL, Erich, A-8061 Weinitzen (AT); LIST, Helmut, A-8010 Graz (AT)
(74) Vertreter: Krause, Walter, Dr. Dipl.-Ing.
(86) Internationale Anmeldenummer: AT9100066
(87) Internationale Veröffentlichungsnummer: WO9118551

(56) Entgegenhaltungen:
- WO-A-89/00397
- US-A- 3 765 402
- US-A- 4 008 717

## Beschreibung

Die Erfindung betrifft eine Vorrichtung zur Entnahme von Körperflüssigkeiten, beispielsweise Blut, Harn oder Gewebeflüssigkeit mit einer Entnahmeeinrichtung, welche mit einer Hohlnadel verbunden ist, sowie einem Speichersystem, mit mehreren separaten auf einer beweglichen Halterung angeordneten Probenbehältern, welche mit einer durchstechbaren Membran gasdicht verschlossen sind, und welche in bestimmten Zeitabschnitten gesammelte Fraktionen der Körperflüssigkeit aufnehmen, wobei eine mit einer Steuereinrichtung verbundene Einrichtung vorgesehen ist, mit welcher die Hohlnadel nacheinander über jeden der Probenbehälter positionierbar und durch die Membran in den Probenbehälter einführbar ist.

Für medizinische Anwendungen besteht häufig der Bedarf der kontinuierlichen oder intermittierenden Gewinnung von biologischen Flüssigkeitin, z.B. Blut, Harn oder auch Gewebeflüssigkeit von Menschen oder Tieren. Vorteilhaft ist es, diese Gewinnung über 24 Stunden, d.h. sowohl bei Tag als auch in den Nachtstunden durchführen zu können, wobei speziell während der Nacht ein ungestörter Schlaf gewährleistet sein soll, um keine Verfälschungen der gemessenen Werte zu bekommen.

Für die beschriebene Anwendung wurde in WO-A-89/00397 eine neue Methode angegeben. Dabei wird über eine Rollenpumpe Blut im Mikroliterbereich, bzw. auch Gewebeflüssigkeit (die durch Einleiten einer Perfusionsflüssigkeit in das Gewebe gewonnen wird) in kapillaren Schläuchen gesammelt, wobei ein Ventilsystem eine zeitliche Zuordnung der Flüssigkeitsproben zu gewissen Entnahmezeitpunkten ermöglicht. Dieses System erfordert einen sehr aufwendigen mechanischen Ventilapparat, was auch mit einem höheren Gewicht des Gerätes verbunden ist. In den einzelnen Leitungsabschnitten des Kapillarsystems kann es zu einem Stillstand der Blutsäule kommen, was Thrombosen innerhalb des Systems begünstigt.

Eine Vorrichtung der eingangs beschriebenen Art ist beispielsweise aus der EP-A-0 223 758 bekanntgeworden. Der hier beschriebene Apparat zur Handhabung kleiner Flüssigkeitsproben weist mehrere Probenbehälter in einer drehbaren Halterung auf, welche über eine Hohlnadel mit Flüssigkeitsproben gefüllt werden können. Die Hohlnadel ist über einen Schlauch mit einer nicht näher beschriebenen Entnahmevorrichtung bzw. Entnahmenadel verbunden, über welche die Probe aufgenommen wird. Eine spezielle Vorrichtung sorgt für die Relativbewegung zwischen Hohlnadel und Probenbehälter, um ein sicheres Einführen in die Probenbehälter zu gewährleisten. Zur Schonung der Nadel ist deren Halterung mit einem Drucksensor ausgestattet, wobei bei axial wirkenden Kräften, welche einen bestimmten Wert überschreiten, eine weitere Bewegung der Nadel unterbunden wird. Bei der Verwendung von Probenbehältern, welche durch eine Membran verschlossen sind, wird auch auf das Problem des sich ändernden Innendruckes bei Probenmanipulationen hingewiesen. Die in der EP-A-0 223 758 angebotene Problemlösung einer Nadel mit zwei Bohrungen, wobei über eine ein Druckausgleich hergestellt werden kann, hat den Nachteil, daß durch diese Öffnung auch Probenteile austreten können. Das Gerät bzw. das Bedienungspersonal, kann dadurch mit der Probe kontaminiert werden.

Aufgabe der Erfindung ist es, ausgehend von einer Vorrichtung der eingangs genannten Art ein kompaktes, auch über längere Zeit am Patienten befestigbares Gerät zu entwickeln, mit welchem unabhängig von der räumlichen Lage des Gerätes Flüssigkeitsproben, wie Blut, Harn und Gewebeflüssigkeit, sicher gesammelt und bis zu deren weiteren Verwendung verwahrt werden können.

Diese Aufgabe wird erfindungsgemäß dadurch gelöst, daß die Entnahmeeinrichtung als zweilumige Kanüle ausgeführt ist, deren ein Lumen über eine erste Verbindungsleitung mit der in die Probenbehälter einführbaren Hohlnadel und deren anderes Lumen über eine zweite Verbindungsleitung mit einer Pumpe verbunden ist, welche von der Steuereinrichtung betätigbar ist, sowie daß die einzelnen Probenbehälter im ungefüllten Zustand eine Gas- oder Gasgemischmenge enthalten, deren Volumen unter Atmosphärendruck kleiner ist als das Volumen der aufzunehmenden Körperflüssigkeit.

In einer ersten, sehr einfachen Ausführungsvariante ist vorgesehen, daß die Probenbehälter starrwandig ausgeführt sind, daß in den Behältern ein im Vergleich zum Umgebungsdruck abgesenkter, eine Pumpwirkung entfaltender Innendruck herrscht, sowie daß die Verbindungsleitung zwischen der zweilumigen Kanüle und der Hohlnadel eine Dosiereinrichtung aufweist. Über die Dosiereinrichtung, beispielsweise ein Dosierventil, kann der gewünschte Flüssigkeitsstrom eingestellt werden. Eine Pumpe für den Flüssigkeitstransport kann bei dieser Ausführungsvariante entfallen, was die Vorrichtung weiter vereinfacht.

Eine andere Ausführungsvariante der Erfindung ist dadurch ausgezeichnet, daß die einzelnen Probenbehälter aus evakuierten, verformbaren Gefäßen bestehen, sowie daß die Verbindungsleitung zwischen der zweilumigen Kanüle und der Hohlnadel eine Pumpe, vorzugsweise eine Schlauchpumpe, zur Forderung der Körperflüssigkeit aufweist.

Um die gewonnenen Flüssigkeitsfraktionen in der Zeit zwischen Entnahme und nachfolgender Auswertung unverfälscht zu lagern, ist es erfindungsgemäß vorgesehen, daß die auf der beweglichen Halterung angeordneten Probenbehälter von einem wärmeisolierenden Gehäuse umgeben sind, dessen Deckel mit Öffnungen für den Durchtritt der Hohlnadel versehen ist, sowie daß im Gehäuse ein mit der Steuereinrichtung verbundener Temperaturfühler und ein Kühlmedium angeordnet sind.

Dabei ist es vorteilhaft, wenn das wärmeisolierende Gehäuse samt den Probenbehältern von der übrigen Vorrichtung abnehmbar ist. Während nun die gesammelten Flüssigkeitsproben im wärmeisolierenden Gehäuse der weiteren Verwendung, z.B. der Auswertung zugeführt werden, kann sofort ein neues Gehäuse mit leeren Probenbehältern an die Vorrichtung angeschlossen und die Probennahme ohne wesentliche Unterbrechung fortgesetzt werden.

Falls die Probennahme in Blutgefäßen erfolgt, ist es von Vorteil, wenn das zu einer Pumpe führende Lumen der zweilumigen Kanüle mit einer Medikamentenpumpe verbunden ist, deren Antrieb mit der Steinereinrichtung in Verbindung steht. Beispeilsweise kann die Medikamentenpumpe Heparin enthalten, wobei das an der Nadelspitze aus einem Lumen austretende Heparin sofort durch das andere Lumen der Nadel abgeführt wird, wodurch nur geringe Heparinmengen in den Körper gelangen und die Blutgerinnung im Leitungssystem der Vorrichtung nachhaltig verhindert wird.

In einer Weiterbildung der Erfindung ist es vorgeshen, daß die Vorrichtung zusätzlich eine weitere Medikamentenpumpe, vorzugsweise eine Kolbenspritze aufweist, deren Antrieb mit der Steuereinrichtung in Verbindung steht. Mit dieser Vorrichtung sind Untersuchungen über Wirkungsweise und zeitliche Konzentrationsverläufe von Medikamenten im Blut möglich. Über die Kolbenspritze kann ein Medikament in genau berechneter Dosierung verabreicht werden, um so aus der Pharmakokinetik dieses Medikamentes Rückschlüsse auf die Körperfunktionen zu gewinnen.

Falls direkt aus dem Gewebe Proben von Gewebeflüssigkeit entnommen werden, ist in einer weiteren Ausführungsvariante der Erfindung vorgesehen, daß die zweite Verbindungsleitung mit einem Behälter für eine Perfusionsflüssigkeit verbunden ist, sowie daß die erste, zur Hohlnadel führende Verbindungleitung in Saugrichtung und die zweite Verbindungsleitung in Pumprichtung über eine Schlauchpumpe geführt ist.

Die Wand des äußeren Lumens der zweilumigen Kanüle kann erfindungsgemäß eine Öffnung, vorzugsweise eine Vielzahl von Öffnungen, aufweisen.

Schließlich ist in einer weiteren Variante der Erfindung vorgesehen, daß die Wand des äußeren Lumens der zweilumigen Kanüle zumindest zum Teil als Diffusionsstrecke ausgebildet ist.

Die Erfindung wird im folgenden anhand von schematischen Zeichnungen näher erläutert. Es zeigen:
Fig. 1 eine erfindungsgemäße Vorrichtung und
Fig. 2 eine Ausführungsvariante der Vorrichtung nach Fig. 1.

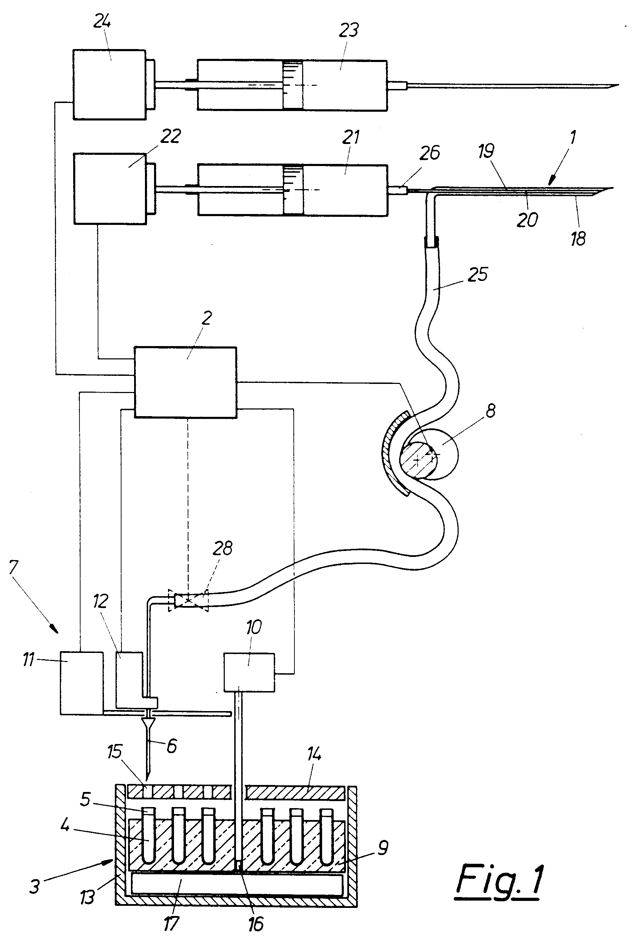

Die in Fig. 1 dargestellte Vorrichtung zur Entnahme von Körperflüssigkeiten weist als Hauptbestandteile eine Entnahmeeinrichtung 1, eine Steuer- bzw. Dateneingabeeinrichtung 2 sowie ein Speichersystem 3 auf. Im Speichersystem 3 sind in mehreren konzentrischen Kreisen Probenbehälter 4 angeordnet, welche mit einer durchstechbaren Membran 5 gasdicht verschlossen sind. Eine mit der Entnahmeeinrichtung 1 über eine Verbindungsleitung verbundene Hohlnadel 6 kann mittels einer mit der Steuereinrichtung 2 verbundenen Einrichtung 7 nacheinander über jeden der Probenbehälter 4 positioniert und durch die Membran 5 in den Probenbehälter 4 eingeführt werden.

Die einzelnen Probenbehälter 4 können entweder starrwandig ausgeführt sein, wobei in den Behältern ein im Vergleich zum Umgebungsdruck abgesenkter Innendruck herrscht, oder aus evakuierten, verformbaren Gefäßen bestehen. In beiden Fällen enthalten die einzelnen Probenbehälter 4 im ungefüllten Zustand eine Gas- oder Gasgemischmenge, deren Volumen unter Atmosphärendruck kleiner ist als das Volumen der aufzunehmenden Körperflüssigkeit. Die Behälter müssen dadurch beim Befüllen nicht entlüftet werden, wodurch das Gerät unabhängig von seiner räumlichen Lage betriebsbereit ist und ein Austreten von Probenbestandteilen verhindert werden kann.

Zwischen der Entnahmeeinrichtung 1 und der Hohlnadel 6 ist eine Dosiereinrichtung angeordnet, welche in Fig. 1 durch eine Schlauchpumpe 8 ralisiert wird. Als Alternative dazu ist bei starrwandigen Probenbehältern 4, welche durch den abgesenkten Innendruck eine Pumpwirkung entfalten, auch ein Dosierventil 28 anstelle der Schlauchpumpe 8 denkbar. Dieses Dosierventil kann auch über die Steuereinrichtung 2 betätigt werden.

Als bewegliche Halterung 9 für die Probenbehälter 4 ist im dargestellten Ausführungsbeispiel ein in einem wärmeisolierenden Gehäuse 13 angeordneter Drehteller vorgesehen, welcher über einen mit der Steuereinrichtung 2 in Verbindung stehenden Antrieb 10 verfügt. Die Einrichtung 7 zur Positionierung der Hohlnadel 6 weist eine erste Antriebsvorrichtung 11 für die Radialbewegung der Hohlnadel 6 sowie eine zweite Antriebsvorrichtung 12, für die Auf- und Abbewegung der Hohlnadel 6 auf. Beide Antriebsvorrichtungen 11 und 12 werden von der Steuereinrichtung 2 mit Signalen beaufschlagt.

Das wärmeisolierende Gehäuse 13 weist einen Verschlußteil bzw. Deckel 14 mit Öffnungen 15 auf, durch welche die Hohlnadel 6 zu den Probenbehältern 4 durchtreten kann. Pro Reihe der in mehreren Reihen angeordneten Probenbehälter ist jeweils nur eine Öffnung 15 vorgesehen, worduch die Kühlverluste gering gehalten werden können.

Im Inneren des Gehäuses 13 ist ein mit der Steuereinrichtung 2 verbundener Temperaturfühler 16 sowie ein Kühlmedium 17 angeordnet. Bei Überschreiten einer zulässigen Maximaltemperatur kann über die Elektronik der Steuereinrichtung 2 ein optischer oder akustischer Alarm ausgelöst werden. Vorteilhafterweise kann das wärmeisolierende Gehäuse 13 samt den Probenbehältern 4 von der übrigen Vorrichtung abgenommen werden. Durch Aufstecken eines neuen Gehäuses mit leeren Probenbehältern kann die Entnahme der Körperflüssigkeit ohne Unterbrechung fortgesetzt werden.

In der Ausführungsvariante nach Fig. 1 ist die in die Probenbehälter 4 einführbare Hohlnadel 6 über eine erste Verbindungsleitung 25 mit dem äußeren Lumen 19 einer als zweilumige Kanüle 18 ausgeführten Entnahmeeinrichtung 1 verbunden, wobei das innere Lumen 20 der Kanüle 18 eine Verbindungsleitung 26 zu einer Medikamentenpumpe, z.B. einer Heparinpumpe 21 aufweist. Der Antrieb 22 der Heparinpumpe steht ebenfalls mit der Steuereinrichtung 2 in Verbindung. Durch die genau dosierbare Heparinmenge, welche an der Spitze der zweilumigen Kanüle 18 austritt und sofort durch den Unterdruck im äußeren Lumen 19 abgeführt wird, wird die Blutgerinnung in der zur Hohlnadel 6 führenden Verbindungsleitung 25 verhindert. Zur Vermeidung der Blutgerinnung ist es aber auch möglich, die Verbindungsleitung 25 mit einer die Thrombosierung verhindernden Substanz zu beschichten.

Die Vorrichtung kann über die Steuereinrichtung 2 auch mit einer weiteren Medikamentenpumpe 23 samt Antrieb 24 in Verbindung stehen. Über die Medikamentenspritze kann laufend ein Medikament in genau gewünschter Dosierung verabreicht werden, um so aus der Pharmakokinetik dieses Medikamentes Rückschlüsse auf die Körperfunktionen zu gewinnen.

Über die Steuer- bzw. Eingabeeinheit 2 hat der Patient außerdem die Möglichkeit, bei Auftreten von unvorhergesehen Ereignissen einen bestimmten Code einzugeben bzw. eine Taste zu drücken. Dadurch wird das Gerät über die Steuerung veranlaßt bestimmte Probenbehälter außerhalb der normalen Reihe zu füllen, welche dann gesondert ausgewertet werden können. Danach kehrt die Vorrichtung automatisch in seinen normalen Betriebszustand zurück.

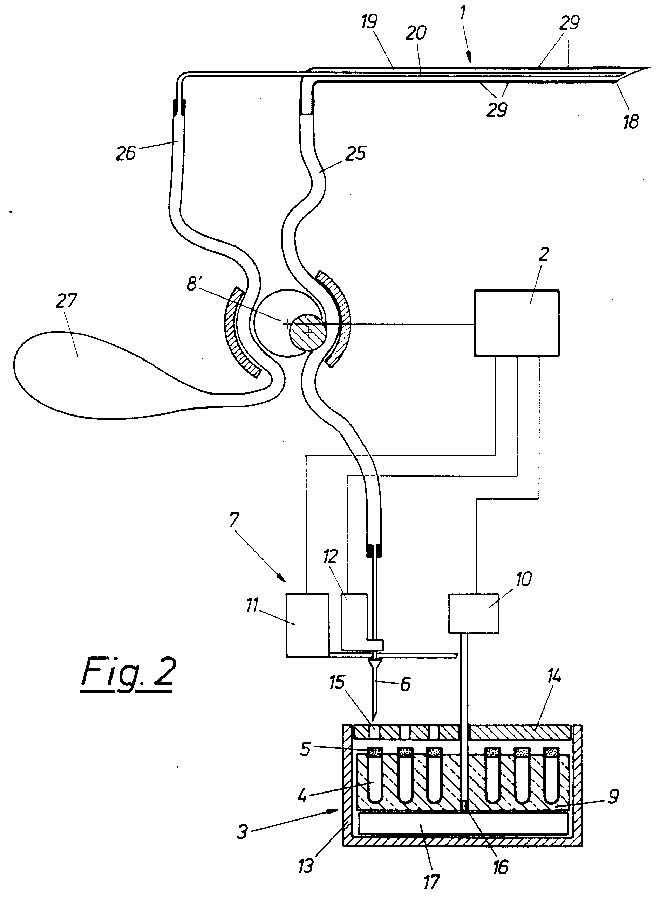

Die Ausführungsvariante nach Fig. 2 eignet sich vor allem für die Gewebeperfusion, bei welcher - wie bereits in der eingangs erwähnten WO-A-89/00397 - eine Perfusionsflüssigkeit in das Gewebe eingebracht und nach einer kurzen Equilibrationsphase wieder abgepumpt wird. Die in die Probenbehälter 4 einführbare Hohlnadel 6 ist hier über die erste Verbindungsleitung 25 mit dem Lumen 19 der zweilumigen Kanüle 18 verbunden, wobei das andere Lumen 20 der Kanüle 18 über die zweite Verbindungsleitung 26 mit einem Behälter 27 für eine Perfusionsflüssigkeit verbunden ist. Die erste Verbindungsleitung 25 kann dabei in Saugrichtung und die zweite Verbindungsleitung 26 in Pumprichtung über eine einzige Schlauchpumpe 8' geführt sein. Die äußere Wand der Kanüle 18 ist mit Öffnungen 29 versehen, welche die Kontaktfläche zum Gewebe vergrößern.

Für besondere Anwendungen kann die Wand des äußeren Lumens 19 der Kanüle 18 auch zum Teil als Diffusionsstrecke ausgebildet sein.

## Patentansprüche

1. Vorrichtung zur Entnahme von Körperflüssigkeiten, beispielsweise Blut, Harn oder Gewebeflüssigkeit mit einer Entnahmeeinrichtung (1), welche mit einer Hohlnadel (6) verbunden ist, sowie einem Speichersystem (3), mit mehreren separaten auf einer beweglichen Halterung (9) angeordneten Probenbehältern (4), welche mit einer durchstechbaren Membran (5) gasdicht verschlossen sind, und welche in bestimmten Zeitabschnitten gesammelte Fraktionen der Körperflüssigkeit aufnehmen, wobei eine mit einer Steureinrichtung (2) verbundene Einrichtung (10, 11, 12) vorgesehen ist, mit welcher die Hohlnadel (6) nacheinander über jeden der Probenbehälter (4) positionierbar und durch die Membran (5) in den Probenbehälter (4) einführbar ist, **dadurch gekennzeichnet**, daß die Entnahmeeinrichtung (1) als zweilumige Kanüle (18) ausgeführt ist, deren ein Lumen (19) über eine erste Verbindungsleitung (25) mit der in die Probenbehälter (4) einführbaren Hohlnadel (6) und deren anderes Lumen (20) über eine zweite Verbindungsleitung (26) mit einer Pumpe (8'; 21) verbunden ist, welche von der Steuereinrichtung (2) betätigbar ist, sowie daß die einzelnen Probenbehälter (4) im ungefüllten Zustand eine Gas- oder Gasgemischmenge enthalten, deren Volumen unter Atmosphärendruck kleiner ist als das Volumen der aufzunehmenden Körperflüssigkeit.

2. Vorrichtung nach Anspruch 1, **dadurch gekennzeichnet**, daß die Probenbehälter (4) starrwandig ausgeführt sind, daß in den Behältern ein im Vergleich zum Umgebungsdruck abgesenkter, eine Pumpwirkung entfaltender Innendruck herrscht, sowie daß die Verbindungsleitung (25) zwischen der zweilumiger Kanüle (18) und der Hohlnadel (6) eine Dosiereinrichtung (8; 28) aufweist.

3. Vorrichtung nach Anspruch 1, **dadurch gekennzeichnet**, daß die einzelnen Probenbehälter (4) aus evakuierten, verformbaren Gefäßen bestehen, sowie daß die Verbindungsleitung (25) zwischen der zweilumigen Kanüle (18) und der Hohlnadel (6) eine Pumpe, vorzugsweise eine Schlauchpumpe (8; 8'), zur Förderung der Korperflüssigkeit aufweist.

4. Vorrichtung nach einem der Ansprüche 1 bis 3, **dadurch gekennzeichnet**, daß die auf der beweglichen Halterung (9) angeordneten Probenbehälter (4) von einem wärmeisolierenden Gehäuse (13) umgeben sind, dessen Deckel (14) mit Öffnungen (15) für den Durchtritt der Hohlnadel (6) versehen ist, sowie daß im Gehäuse (13) ein mit der Steuereinrichtung (2) verbundener Temperaturfühler (16) und ein Kühlmedium (17) angeordnet sind.

5. Vorrichtung nach Anspruch 4, **dadurch gekennzeichnet**, daß das wärmeisolierende Gehäuse (13) samt den Probenbehältern (4) von der übrigen Vorrichtung abnehmbar ist.

6. Vorrichtung nach einem der Ansprüche 1 bis 5, **dadurch gekennzeichnet**, jdaß das zu einer Pumpe führende Lumen (20) der zweilumigen Kanüle (18) mit einer Medikamentenpümpe (21) verbunden ist, deren Antrieb (22) mit der Steuereinrichtung (2) in Verbindung steht.

7. Vorrichtung nach Anspruch 6, **dadurch gekennzeichnet**, daß die Vorrichtung zusätzlich eine weitere Medikamentenpumpe (23), vorzugsweise eine Kolbenspritze aufweist, deren Antrieb (24) mit der Steuereinrichtung (2) in Verbindung steht.

8. Vorrichtung nach einem der Ansprüche 1 bis 5, **dadurch gekennzeichnet**, daß die zweite Verbindungsleitung (26) mit einem Behälter (27) für eine Perfusionsflüssigkeit verbunden ist, sowie daß die erste, zur Hohlnadel (6) führende Verbindungleitung (25) in Saugrichtung und die zweite Verbindungsleitung (26) in Pumprichtung über eine Schlauchpumpe (8') geführt ist.

9. Vorrichtung nach Anspruch 8, **dadurch gekennzeichnet**, daß die Wand des äußeren Lumens (19) der zweilumigen Kanüle (18) zumindest zum Teil als Diffusionsstrecke aus gebildet ist.

10. Vorrichtung nach Anspruch 8, **dadurch gekennzeichnet**, daß die Wand des äußeren Lumens (19) der zweilumigen Kanüle (18) zumindest eine Öffnung (29), vorzugsweise eine Vielzahl von Öffnungen (29), aufweist.

## Claims

1. A device for the sampling of body fluids, such as blood, urine, or tissue fluid, comprising a sampling unit (1) connected to a hollow needle (6), and a storage system (3) with several separate sample containers (4) placed on a movable carrier (9), which are sealed so as to be gas-tight by a puncturable membrane (5), and which are intended to receive collected fractions of the body fluid at given intervals, a device (10, 11, 12) connected to a control unit (2) being provided, which will permit the hollow needle (6) to be positioned over each sample container (4) in turn and to be inserted into the sample container (4) through the membrane (5), wherein the sampling device (1) is configured as a two-channel cannula (18), one of whose channels (19) is connected via a first connecting tube (25) to the hollow needle (6) used for insertion into the sample containers (4), while the other channel (20) is connected via a second connecting tube (26) to a pump (8'; 21) operated by the control unit (2), and wherein the individual sample containers (4) contain an amount of gas or gas mixture before use, whose volume is smaller at atmospheric pressure than the volume of the body fluid to be received.

2. A device according to claim 1, wherein the sample containers (4) have rigid walls, and wherein the pressure prevailing inside the containers is lower than the ambient pressure, thus developing a pumping effect, and wherein the connecting tube (25) between the two-channel cannula (18) and the hollow needle (6) is provided with a metering unit (8; 28).

3. A device according to claim 1, wherein the individual sample containers (4) are configured as evacuated, deformable vessels, and wherein the connecting tube (25) between the two-channel cannula (18) and the hollow needle (6) is provided with a pump, preferably a peristaltic pump (8; 8'), for handling the body fluid.

4. A device according to any of claims 1 to 3, wherein the sample containers (4) placed on the movable carrier (9) are encased by a heat-insulating housing (13) with openings (15) in its cover (14) for the passage of the hollow needle (6), and wherein the housing (13) contains a temperature sensor (16) connected to the control unit (2) as well as a cooling medium (17).

5. A device according to claim 4, wherein the heat-insulating housing (13) including the sample containers (4) is detachable from the remaining device.

6. A device according to any of claims 1 to 5, wherein the channel (20) of the two-channel cannula (18), which is leading to a pump, is connected to a drug pump (21), whose drive (22) is connected to the control unit (2).

7. A device according to claim 6, wherein the device is furnished with an additional drug pump (23), preferably a syringe, whose drive (24) is in connection with the control unit (2).

8. A device according to any of claims 1 to 5, wherein the second connecting tube (26) leads into a container (27) for a perfusion fluid, and wherein the first connecting tube (25), which leads to the hollow needle (6), is passed through a peristaltic pump (8') in the direction of suction, while the second connecting tube (26) is passed through this pump (8') in pumping direction.

9. A device according to claim 8, wherein the wall of the outer channel (19) of the two-channel cannula (18) is configured, at least in part, as a diffusion path.

10. A device according to claim 8, wherein the wall of the outer channel (19) of the two-channel cannula (18) has a perforation (29), preferably a number of perforations.

## Revendications

1. Dispositif pour le prélèvement de liquides corporels, par exemple du sang, de l'urée ou du liquide de tissus, avec un dispositif de prélèvement (1) qui est relié à une aiguille creuse (6), ainsi qu'un système de stockage (3) à plusieurs récipients d'échantillons (4) disposés séparément sur un support (9) mobile, qui sont bouchés de manière étanche aux gaz par une membrane (5) pouvant être percée et qui reçoivent à intervalles de temps déterminés des fractions réunies du liquide corporel, un dispositif (10, 11, 12) relié à un organe de commande (2) étant prévu pour permettre de positionner l'aiguille creuse (6) au-dessus de chacun des récipients d'échantillons (4) les uns après les autres, et de l'introduire à travers la membrane (5) dans les récipients d'échantillons (4), caractérisé en ce que le dispositif de prélèvement (1) est réalisé comme une canule à deux lumières (18) dont une lumière (19) est reliée par une première conduite de liaison (25) à l'aiguille creuse (6) à introduire dans les récipients d'échantillons (4) et dont l'autre lumière (20) est reliée par une deuxième conduite de liaison (26) à une pompe (8', 21) qui peut être actionnée par l'organe de commande (2), et en ce que les différents récipients d'échantillons (4) contiennent, à l'état non rempli, une quantité de gaz ou de mélange gazeux dont le volume à la pression atmosphérique est plus petit que le volume du liquide corporel à recevoir.

2. Dispositif selon la revendication 1, caractérisé en ce que dans les récipients règne une pression intérieure diminuée comparativement à la pression ambiante, en exerçant ainsi un effet de pompage, et en ce que la conduite de liaison (25) comporte, entre la canule (18) à deux lumières et l'aiguille creuse (6), un dispositif de dosage (8 ; 28).

3. Dispositif selon la revendication 1, caractérisé en ce que les différents récipients d'échantillons (4) sont constitués de récipients où on a fait le vide et qui sont déformables, et aussi, en ce que la conduite de liaison (25) comprend, entre la canule (18) à deux lumières et l'aiguille creuse (6), une pompe de préférence tubulaire (8, 8') pour le transport du liquide corporel.

4. Dispositif selon l'une des revendications 1 à 3, caractérisé en ce que les récipients d'échantillons (4) disposés sur le support mobile (9) sont entourés d'un boîtier (13) à isolation thermique dont le couvercle (14) est muni d'ouvertures (15) pour le passage de l'aiguille creuse (6), et aussi en ce que dans le boîtier (13) sont placés un capteur de température (16) relié à l'organe de commande (2) et un milieu refroidisseur (17).

5. Dispositif selon la revendication 4, caractérisé en ce que le boîtier (13) à isolation thermique contenant les récipients d'échantillons (4) peut être enlevé du reste du dispositif.

6. Dispositif selon l'une des revendications 1 à 5, caractérisé en ce que la lumière (20) de la canule (18) à deux lumières, conduisant à une pompe, est liée à une pompe pour médicaments (21) dont le dispositif moteur (22) est en liaison avec l'organe de commande (2).

7. Dispositif selon la revendication 6, caractérisé en ce que le dispositif comprend additionnellement une autre pompe pour médicaments (23), de préférence une seringue à piston, dont le dispositif moteur (24) est en liaison avec l'organe de commande (2).

8. Dispositif selon l'une des revendications 1 à 5, caractérisé en ce que la deuxième conduite de liaison (26) est reliée à un récipient (27) destiné à un liquide de perfusion, et aussi en ce que la première conduite de liaison (25) menant à l'aiguille creuse (6) est commandée dans le sens de l'aspiration et la deuxième conduite de liaison dans le sens du pompage, par l'intermédiaire d'une pompe tubulaire (8').

9. Dispositif selon la revendication 8, caractérisé en ce que la paroi de la lumière extérieure (19) de la canule (18) à deux lumières est réalisée au moins partiellement comme chemin de diffusion.

10. Dispositif selon la revendication 8, caractérisé en ce que la paroi de la lumière extérieure (19) de la canule (18) à deux lumières comporte au moins une ouverture (29) et de préférence un grand nombre d'ouvertures (29).
